Europäisches Patentamt

European Patent Office

Office européen des brevets

(19)

(11) Veröffentlichungsnummer: **0 033 540**
·B1

(12) **EUROPÄISCHE PATENTSCHRIFT**

(45) Veröffentlichungstag der Patentschrift:
24.10.84

(21) Anmeldenummer: 81100738.4

(22) Anmeldetag: 02.02.81

(51) Int. Cl.³: **C 12 N 9/04, C 12 Q 1/26**

(54) Verfahren und Reagens zur Bestimmung von Glycerin.

(30) Priorität: 05.02.80 DE 3004129

(43) Veröffentlichungstag der Anmeldung:
12.08.81 Patentblatt 81/32

(45) Bekanntmachung des Hinweises auf die Patenterteilung:
24.10.84 Patentblatt 84/43

(84) Benannte Vertragsstaaten:
AT BE CH DE FR GB IT LI LU NL SE

(56) Entgegenhaltungen:
EP - A - 0 010 296
DE - A - 2 817 087
GB - A - 2 025 979
US - A - 4 125 377

CHEMICAL ABSTRACTS, Band 92, Nr. 11, 17. März 1980,
Nr. 89786f, Seite 203, COLUMBUS OHIO (US)
AGRICULTURAL & BIOLOGICAL CHEMISTRY, vol. 44,
no. 2, February 1980, TOKYO (JP), T. UWAJIMA et al.:
"Formation and purification of a new enzyme glycerol
oxidase and stoichiometry of the enzyme reaction",
Seiten 399-406

(73) Patentinhaber: BOEHRINGER MANNHEIM GMBH,
Patentabteilung, Abt. E Sandhofer
Strasse 112-132 Postfach 31 01 20,
D-6800 Mannheim 31 Waldhof (DE)

(72) Erfinder: Gauhl, Heimgard, Kustermannstrasse 31,
D-8132 Tutzing (DE)
Erfinder: Seidel, Hans, Dr., Waxensteinstrasse 6,
D-8132 Tutzing (DE)
Erfinder: Lang, Gunter, Lange Strasse 22,
D-8132 Tutzing (DE)
Erfinder: Röder, Albert, Dr., Bahnhofstrasse 41,
D-8124 Seeshaupt (DE)
Erfinder: Ziegenhorn, Joachim, Dr., Ina-Seidel-Weg 1,
D-8130 Starnberg (DE)

(74) Vertreter: Weickmann, Heinrich, Dipl.-Ing. et al,
Patentanwälte Dipl.-Ing. H.Weickmann Dipl.-Phys.Dr.
K.Fincke Dipl.-Ing. F.A.Weickmann Dipl.-Chem. B. Huber
Dr.-Ing. H. Liska Dipl.-Phys.Dr. J. Prechtel
Postfach 860820, D-8000 München 86 (DE)

EP 0 033 540 B1

## Beschreibung

Die Erfindung betrifft ein Verfahren und ein Reagens zur enzymatischen Bestimmung von Glycerin mittels einer bestimmten Glycerinoxidase, diese selbst und ihre Gewinnung.

Die Bestimmung von Triglyceriden ( = Glycerinester von langkettigen Fettsäuren) hat für die medizinische Diagnostik eine erhebliche Bedeutung. Ein erhöhter Triglyceridspiegel im Blut ist ein wesentlicher Risikofaktor der Arteriosklerose. Bei hohen Triglyceridwerten, also Hypertriglyceridämie, kommen Coronarinsuffizienz und Herzinfarkt häufiger vor als bei niedrigen Triglyceridwerten. Hypertriglyceridämie begünstigt das Auftreten von Arteriosklerose und Coronarerkrankungen und muss daher frühzeitig erkannt werden, damit die Behandlung rechtzeitig beginnen kann. Eine rasche und zuverlässig durchführbare Methode zur Bestimmung von Triglyceriden bzw. Glycerin hat daher grosse Bedeutung.

Die bekannten und brauchbaren Methoden zur Triglyceridbestimmung basieren auf der enzymatischen Hydrolyse der Triglyceride mittels Lipase/Esterase und Bestimmung des freigesetzten Glycerins, beispielsweise mittels Glycerokinase/Pyruvatkinase/Lactatdehydrogenase oder mittels Glycerinoxidase (DE-AS 2 817 087).

Diese bekannten Verfahren weisen jedoch wesentliche Nachteile auf. So ist bei der Glycerokinasemethode die Haltbarkeit des Reagens infolge Anwesenheit zahlreicher empfindlicher Coenzyme und Enzyme gering und es müssen stets Probenleerwert-Bestimmungen durchgeführt werden. Bei der bekannten Glycerinoxidase-Methode liegt ein wesentlicher Nachteil darin, dass das Enzym in den bekannten, dieses enthaltende Mikroorganismen in recht geringen Mengen vorliegt und zudem eine niedrige spezifische Aktivität in der Grössenordnung von 30 U/mg aufweist.

Aufgabe der Erfindung ist daher die Schaffung eines neuen Verfahrens und eines Reagens zur Bestimmung von Triglyceriden bzw. Glycerin, welches die Nachteile der bisher bekannten Glycerinoxidase-Methode nicht aufweist, deren Vorteile jedoch beibehält.

Gelöst wird diese Aufgabe erfindungsgemäss durch ein Verfahren zur Bestimmung von Glycerin durch Oxidation mit Sauerstoff in Gegenwart von Glycerinoxidase und Messung des Sauerstoffverbrauchs oder der $H_2O_2$-Bildung, welches dadurch gekennzeichnet ist, dass man eine Glycerinoxidase aus Aspergillus spec. DSM 1729 verwendet.

Die Erfindung beruht auf der überraschenden Auffindung des Mikroorganismus Aspergillus spec. DSM 1729, welcher nicht nur einen um mehrere Zehnerpotenzen höheren Gehalt an Glycerinoxidase aufweist im Vergleich zu bekannten glycerinoxidasehaltigen Mikroorganismen, sondern darüber hinaus auch eine Glycerinoxidase mit wesentlich höherer spezifischer Aktivität enthält.

Im Rahmen des erfindungsgemässen Verfahrens kann der Sauerstoffverbrauch oder gebildetes $H_2O_2$ nach hierfür an sich bekannten Methoden bestimmt werden. Vorzugsweise wird der Sauerstoffverbrauch polarometrisch bestimmt mittels Sauerstoffelektrode, da sich dieses Verfahren besonders zur automatischen Durchführung eignet. Besonders geeignet sind hierfür die in den DE-OSen 2 130 340 und 2 130 308 beschriebenen Methoden zur polarometrischen Messung des Sauerstoffverbrauchs in wässrigem Medium. Ein anderes geeignetes Verfahren ist die Gaschromatographie.

Gebildetes $H_2O_2$ kann sowohl titrimetrisch als auch potentiometrisch, polarographisch und kolorimetrisch sowie enzymatisch bestimmt werden. Bevorzugt werden die enzymatischen Methoden unter Verwendung von Katalase und Peroxidase, da diese nicht nur äusserst spezifisch und zuverlässig sind, sondern auch mit der Hauptreaktion unter Bildung von Wasserstoffperoxid auf einfachste Weise kombiniert werden können. Als besonders geeignet erwies sich insbesondere die Bestimmung mittels Katalase in Gegenwart von β-Diketonen, z.B. Acetylaceton und Methanol bzw. Äthanol oder Methylenglykol sowie die Bestimmung mittels Peroxidase in Gegenwart eines oder mehrerer Chromogene. Bei der Bestimmung mittels Katalase, Acetylaceton und Methanol wird letzteres zu Formaldehyd oxidiert, der mit Acetylaceton eine Farbreaktion eingeht, die gemessen werden kann. Bei der Bestimmung mittels Peroxidase werden als Chromogen Verbindungen eingesetzt, die nach der Reaktion photometrisch bestimmt werden können.

Ein Beispiel für ein geeignetes Chromogen ist 2,2'-Aminobenzthiazolin-sulfonsäure. Ein anderes bevorzugtes Beispiel ist das Indikatorsystem nach Trinder [Ann. Clin. Biochem. 6 (1969), 24–27], bei dem Phenol mit 4-Aminoantipyrin (4-AAP) in Gegenwart von POD und unter der Einwirkung von $H_2O_2$ oxidativ zu einem Farbstoff gekuppelt werden. Anstelle von Phenol können Phenolderivate, Anilinderivate, Naphthol, Naphtholderivate, Naphthylamin, Naphthylaminderivate, Aminochinoline, Hydroxychinoline, Dihydroxyphenylessigsäure und ähnlich reagierende Substanzen eingesetzt werden. Anstelle von 4-Aminoantipyrin können 4-Aminoantipyrin-Derivate, Phenylendiaminsulfonsäure, MBTH (Methylbenzothiazolonhydrazon), S-MBTH (sulfoniertes Methylbenzothiazolonhydrazon MBTH- und S-MBTH-Derivate sowie ähnlich reagierende Verbindungen eingesetzt werden.

Gegenstand der Erfindung ist weiter ein Reagens zur Bestimmung von Glycerin, welches dadurch gekennzeichnet ist, dass es aus Glycerinoxidase aus Aspergillus spec. DSM 1729 und einem System zur Bestimmung von $H_2O_2$ besteht. Vorzugsweise enthält dieses Reagens zusätzlich ein Mittel zur Verseifung von verestertem Glycerin, insbesondere Lipase/Esterase oder Esterase. Hiefür geeignete Reagenzien sind dem Fachmann bekannt und brauchen nicht weiter beschrieben zu werden.

In einer bevorzugten Ausführungsform besteht das erfindungsgemässe Reagens im wesentlichen aus Glycerinoxidase, Katalase, Acetylaceton, Methanol und Puffer, einzeln oder gemischt. In einer weiteren bevorzugten Ausführungsform besteht das Reagens im wesentlichen aus Glycerinoxidase, Peroxidase, wenigstens einem Chromogen und Puffer, einzeln oder gemischt. Unter Glycerinoxidase wird hier und im folgenden stets Glycerinoxidase aus Aspergillus spec. DSM 1729 verstanden. Als Farbindikatorsystem wird das System nach Trinder bevorzugt.

Die oben erwähnten bevorzugten Reagenskombinationen können ausser den aufgeführten obligaten Bestandteilen zusätzlich übliche Lösungsmittel, Stabilisatoren oder/und oberflächenaktive Substanzen enthalten. Alle diese Zusatzstoffe sind dem Fachmann bekannt und in Nachweissystemen für Wasserstoffperoxid üblich. Als Puffer eignen sich zwischen pH 5 und pH 10, vorzugsweise pH 6 und pH 9, puffernde Substanzen. Typische Beispiele für geeignete Puffer sind Phosphatpuffer, Tris-Puffer, TRA-Puffer, Acetatpuffer, Citratpuffer und Hepes-Puffer (N-2-Hydroxyäthylpiperazin-N-äthan-sulfonsäure).

Vorzugsweise enthalten die oben erwähnten Reagenskombinationen die essentiellen Bestandteile in folgenden Mengenverhältnissen:
2 bis 150 U/ml Glycerinoxidase,
0,5 bis 100 U/ml Peroxidase,
0,05 bis 20 mMol/l wenigstens eines Chromogens sowie
0,001 bis 0,1 g/ml wenigstens eines oberflächenaktiven Mittels in Puffer, pH 6 bis 9.

Falls die Reagenskombination für die kinetische Bestimmung verwendet werden soll, werden folgende Mengenverhältnisse bevorzugt:
100 bis 250 KU/l Glycerinoxidase,
5 bis 50 KU/l Peroxidase,
0,2 bis 5 mMol/l Chromogen und gegebenenfalls 1 bis 5 g/l wenigstens eines oberflächenaktiven Mittels oder ein Vielfaches davon, und Puffer pH 6,8 bis 8,0.

Die Züchtung des für die Gewinnung der Glycerinoxidase verwendeten Mikroorganismus Aspergillus spec. DSM 1729 erfolgt nach an sich bekannten Methoden unter Verwendung eines Nährmediums, welches Glycerin, Malzextrakt und Hefeextrakt als wesentliche Bestandteile neben Puffer, Salzen und Spurenelementen enthält. Für die Vorkultur verwendet man vorzugsweise ein Medium mit 1 bis 100 g/l Glycerin, 1 bis 50 g/l Malzextrakt, 1 bis 10 g/l Hefeextrakt, 1 bis 100 g/l Baumwollsamenmehl sowie 0,2 bis 5 g/l $K_2HPO_4 \cdot 3H_2O$ (oder eine äquivalente Menge eines anderen $K_2HPO_4$-Präparats), 0,2 bis 5 g/l $KNO_3$, 0,1 bis 5 g/l $CaCO_3$, 0,1 bis 1 g/l NaCl, 0,001 bis 0,1 g/l $FeSO_4$ (als Heptahydrat gemessen) und 0,1 bis 5 g/l $MgSO_4$ (als Heptahydrat gemessen).

Für die Hauptkultur wird vorzugsweise ein Medium mit 20 bis 200 g/l Glycerin, 5 bis 100 g/l Malzextrakt, 0,5 bis 5 g/l Hefeextrakt, 0,2 bis 5 g/l Dikaliumphosphat, 0,2 bis 5 g/l $KNO_3$, 0,1 bis 5 g/l $CaCO_3$, 0,1 bis 1 g/l NaCl, 0,001 bis 0,1 g/l Ferrosulfat und 0,1 bis 5 g/l Magnesiumsulfat verwendet. Für die einzelnen Salze gilt das oben zur Vorkultur Ausgeführte entsprechend.

Die Vorkultur wird zweckmässigerweise als Schüttelkultur unter Belüftung bei Temperaturen zwischen 25 und 40 °C durchgeführt. Die Kulturdauer liegt im allgemeinen zwischen 20 und 60 Stunden.

Für die Hauptkultur gilt Entsprechendes hinsichtlich Temperatur und Züchtungsdauer. Im Kleinfermenter werden besonders gute Ergebnisse bei Zufuhr von 0,5 bis 1 l Luft/Minute/l Medium erzielt.

Nach Beendigung der Züchtung wird die Biomasse in üblicher Weise abgetrennt und die Zellen werden aufgeschlossen. Die üblichen Aufschlussmethoden können angewendet werden, bevorzugt wird mechanisch aufgeschlossen. Aus der Aufschlusslösung werden die unlöslichen Teile entfernt, z.B. durch Abzentrifugieren oder Abnutschen. Die erhaltene Lösung kann als solche bereits direkt für die Glycerinbestimmung eingesetzt werden. Vorzugsweise erfolgt jedoch eine weitere Aufreinigung des Enzyms.

Die Aufreinigung kann nach üblichen biochemischen Methoden der Enzymfraktionierung erfolgen. Als besonders geeignet erwies sich jedoch eine in der Enzymfraktionierung unübliche Stufe, nämlich eine Fällung mit Trichloressigsäure. Trichloressigsäurezusatz wird üblicherweise nur zur vollständigen Fällung von Proteinen, also zur Eiweissentfernung eingesetzt, wenn Interesse lediglich an der eiweissfreien Lösung besteht. Überraschenderweise stellte sich jedoch heraus, dass das erfindungsgemässe Enzym durch Trichloressigsäure ohne Aktivitätsverlust und mit hoher Anreicherungswirksamkeit gefällt werden kann. Die Fällung erfolgt dabei zweckmässig durch Zusatz der Trichloressigsäure bis auf einen pH-Wert im Bereich von 5,4 bis 4,6. Im abgetrennten Niederschlag findet sich das gewünschte Enzym mit einer spezifischen Aktivität von etwa 300 U/mg.

Falls eine weitere Reinigung gewünscht wird, erfolgt diese zweckmässig durch Acetonfällung, anschliessende Ammonsulfatfraktionierung sowie abschliessende Behandlung mit einem schwach basischen Austauscherharz. Bei der Acetonfällung setzt man der Lösung zweckmässig 0,25 bis 0,35 Volumen Aceton zu. Die Ammonsulfatfraktionierung wird zweckmässig bei einer Molarität von 0,5 bis 2,0 durchgeführt. Sie führt bereits zu einer spezifischen Aktivität von mehr als 800 U/mg. Die Behandlung mit einem schwach basischen Ionenaustauscherharz, vorzugsweise mit Diäthylaminoäthanolgruppen-haltigem vernetztem Dextran (DEAE Sephadex) ergibt nochmals eine 3- bis 10-fache Aufreinigung und führt zu einer Glycerinoxidase mit einer spezifischen Aktivität von etwa 2500 bis 8000 U/mg.

Da mit dem erfindungsgemäss eingesetzten Mikroorganismus bereits Aktivitäten von 150 000 bis 200 000 U/l Kultur erreicht werden, lässt sich auf diese Weise die erforderliche Glycerinoxidasemenge für die Glycerinbestimmung drastisch absenken und die Herstellung entscheidend verbilligen.

Die oben beschriebene Züchtungs- und Reinigungsmethode setzt voraus, dass das Enzym während der Züchtung nicht an das Medium abgegeben wird. Durch Züchtung in Gegenwart von Detergentien lässt sich jedoch wenigstens ein Teil der Aktivität in das Medium überführen. Ebenso ist eine Extraktion aus den geernteten Zellen mit Detergentien möglich. Die Ergebnisse stehen jedoch hinter denen des mechanischen Aufschlusses, beispielsweise in der Hochdruckdispersion, zurück.

Das erfindungsgemässe Enzym unterscheidet sich von bisher bekannten Glycerinoxidasen vor allem durch ein wesentlich niedrigeres Molekulargewicht. Während beispielsweise die bisher für die Glycerinbestimmung verwendete, Glycerinaldehyd bildende Glycerinoxidase ein Molekulargewicht von mindestens 300 000 aufweist, beträgt letzteres beim erfindungsgemässen Enzym nur etwa 90 000. Ferner wird die Aktivität des erfindungsgemässen Enzyms durch Kupfersulfat oder Bleiacetat nicht gehemmt, während SH-Reagenzien inhibieren. Bei der erwähnten bekannten Glycerinoxidase dagegen stabilisieren SH-Reagenzien und Kupfersulfat und Bleiacetat hemmen die Aktivität zu etwa 90%. Weitere wesentliche Unterschiede bestehen in einer spezifischen Aktivität zwischen 2000 und 8000 U/mg beim erfindungsgemässen Enzym gegenüber etwa 30 U/mg beim bekannten Enzym. Auch ist die Stabilität des erfindungsgemässen Enzyms bei pH-Werten von 5,0 bzw. 10,0 wesentlich besser und die Aktivität nach 10 Minuten bei 37 °C beträgt unter diesen Bedingungen noch 50 bis 55% gegenüber 6 bzw. 3% beim bekannten Enzym.

Das Verfahren und das Reagens der Erfindung eignen sich zur Bestimmung von Glycerin bzw. Glycerinestern (Triglyceriden) in wässrigen Medien aller Art, wie Lebensmittelextrakten, Körperflüssigkeiten und insbesondere Serum. Aufgrund der grossen Spezifität des Verfahrens wird nur freies Glycerin bestimmt. Die Bestimmung des in erster Linie interessierenden veresterten Glycerins lässt sich nach Verseifung des letzteren durchführen. Die Verseifung erfolgt vorzugsweise enzymatisch, da sie dann gleichzeitig mit der eigentlichen Glycerinbestimmung erfolgen kann.

Die folgenden Beispiele erläutern die Erfindung weiter.

Beispiel 1

1) Züchtung des Mikroorganismus

60 ml einer Vorkultur, enthaltend 10 g/l Glycerin, 10 g/l Malzextrakt, 5 g/l Hefeextrakt, 10 g/l Baumwollsamenmehl, 1,5 g/l $K_2PO_4 \cdot 3H_2O$, 1 g/l $KNO_3$, 2,0 g/l $CaCO_3$, 0,5 g/l NaCl, 0,01 g/l $FeSO_4 \cdot 7H_2O$ und 1,0 g/l $MgSO_4 \cdot 7H_2O$ wurden mit Sporen von Aspergillus spec. DSM 1729 beimpft und 48 Stunden bei 30 °C unter Luftzufuhr geschüttelt.

360 ml der so erhaltenen Vorkultur wurden in 15 l eines Hauptkulturmediums eingebracht, welches 100 g/l Glycerin, 40 g/l Malzextrakt, 2,5 g/l Hefeextrakt, 1,5 g/l $K_2HPO_4 \cdot 3H_2O$, 1,0 g/l $KNO_3$, 2,0 g/l $CaCO_3$, 0,5 g/l NaCl, 0,01 g/l $FeSO_4 \cdot 7H_2O$ und 1,0 g/l $MgSO_4 \cdot 7H_2O$ enthielt. In einem 25-l-Fermenter, der mit 600 Upm gerührt und mit 10 l Luft/Minute belüftet wurde, erfolgte dann bei 30 °C die Hauptkultur. Nach 22 Stunden Züchtungsdauer betrug die Aktivität 174 800 U/l. Die Aktivitäsmessung erfolgte in 0,1 M TRA-Puffer, pH 8, bei 25 °C mit p-Chlorphenol und 4-Aminoantipyrin und 546 nm.

2) Reinigung des Enzyms

100 g der wie oben beschrieben erhaltenen Trockenmasse werden in 5 l 0,02 M Acetatpuffer, pH 6,0, suspendiert und über Hochdruckdispersion aufgeschlossen. Anschliessend wird zentrifugiert und der Niederschlag verworfen.

Dem Überstand wird eine 0,1 molare Trichloressigsäurelösung zugefügt, bis ein pH-Wert von 4,8 erreicht ist. Der Niederschlag wird abzentrifugiert. Er weist eine spezifische Aktivität von 300 U/mg auf.

3) Feinreinigung

Der Niederschlag der Trichloressigsäurefällung wird mit 500 ml 0,1 M Acetatpuffer, pH 6,0, gelöst und mit 0,3 Volumen Aceton bei 0 °C versetzt. Der gebildete Niederschlag wird abzentrifugiert und erneut mit 0,1 M Acetatpuffer, pH 6,0, gelöst. Der so gewonnenen Enzymlösung wird Ammoniumsulfat bis zu einer Molarität von 1,0 zugesetzt. Der Niederschlag wird abzentrifugiert. Seine spezifische Aktivität beträgt ca. 800 U/mg.

Der Niederschlag wird erneut im gleichen Puffer wie bei der Ammonsulfatfraktionierung gelöst, gegen gleichen Puffer dialysiert und dann mit DEAE-Sephadex (äquilibriert gegen den gleichen Puffer) versetzt und eine Stunde gerührt. Dann wird das Austauscherherz mit gleichem Puffer gewaschen und mit 0,2 M Acetatpuffer, pH 6,0, fraktioniert eluiert. Die aktiven Fraktionen werden vereinigt. Die spezifische Aktivität des so erhaltenen Produkts liegt in den einzelnen Fraktionen zwischen 2500 und 8000 U/mg.

Beispiel 2

Bestimmung von Glycerin über $H_2O_2$-Bildung

Es werden zwei Reagenzien hergestellt:

Reagens 1: 0,1 Mol/l Triäthanolamin/HCl-Puffer, pH 8,0

3,6 mMol/l bzw. 2 g/l Isotridecyläther

4,7 mMol/l bzw. 2 g/l Natriumcholat 10 mMol/l p-Chlorphenol

0,5 mMol/l aminosubstituiertes 4-Aminoantipyrin

10 U/ml Peroxidase.

Reagens 2: 500 U/ml Glycerinoxidase.

Zur Durchführung der Bestimmung werden 2 ml Reagens 1 und 0,2 ml Reagens 2 in eine Küvette pipettiert. Die Extinktion $E_1$ wird am Photometer bei 546 nm abgelesen. Anschliessend wird die Reaktion durch Zugabe von 20 µl Probe gestartet. Zur Bestimmung von freiem Glycerin können wässrige Medien aller Art, wie Lebensmittelextrakte, Körperflüssigkeiten und insbesondere Serum als Probe eingesetzt werden, zur Bestimmung von Triglyceriden nach Verseifung.

Nach 20 Minuten Reaktionszeit wird die Extinktion $E_2$ abgelesen. Die Inkubationstemperatur beträgt 25 °C.

Die Auswertung erfolgt über eine Eichgerade, bei der die gemessene Extinktionsdifferenz

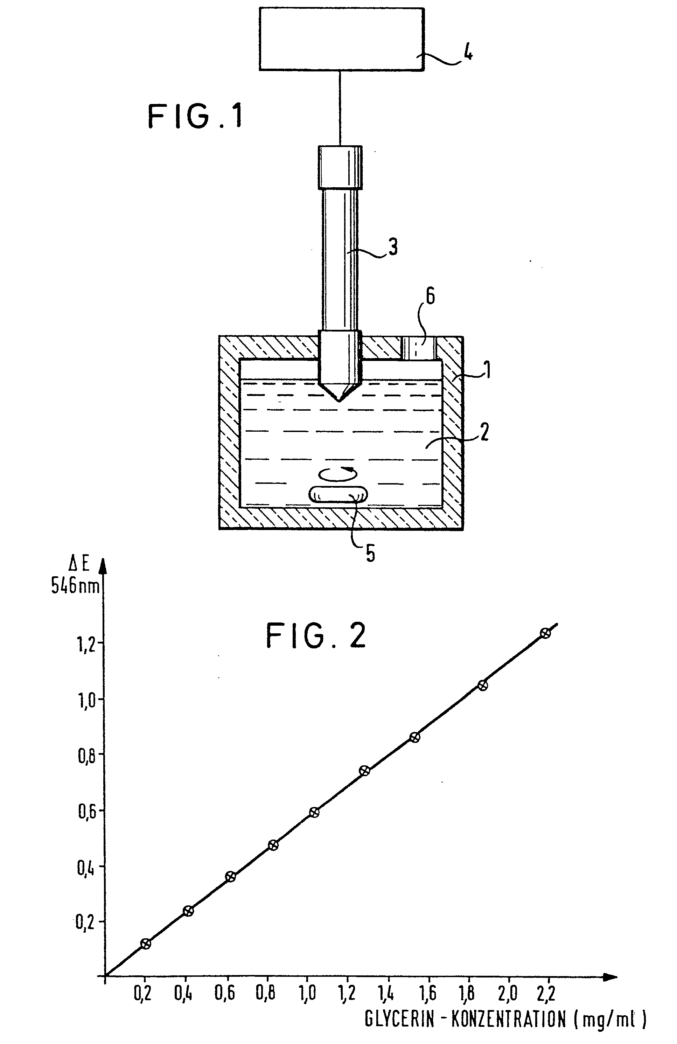

$\Delta E = E_2 - E_1$ einer Glycerinstandardlösung zur Glycerin- bzw. Triglyceridkonzentration in Beziehung gesetzt wird (Fig. 2 der Zeichnung).

Die Konzentrationen im Testansatz betragen:
0,09 Mol/l Triäthanolamin/HCl-Puffer, pH 8,0
1,8 g/l (3,2 mMol/l) Isotridecyläther
4,3 mMol/l Natriumcholat
9 mMol/l p-Chlorphenol
0,45 mMol/l aminosubstituiertes 4-Aminoantipyrin
9 U/ml Peroxidase
45 U/ml Glycerinoxidase.

Beispiel 3
Bestimmung des Glycerins über den Sauerstoffverbrauch

Es wird die in Fig. 1 der Zeichnung dargestellte Vorrichtung verwendet. Die Vorrichtung besteht aus einem Reaktionsgefäss 1 in Form einer zylindrischen Kammer aus transparentem Kunststoff mit einem Innendurchmesser von 143 mm und einer Innenhöhe von 220 mm. Die Kammer enthält 1,8 ml einer Lösung von 18 mM Kaliumjodid, 7,5 mM Ammoniumheptamolybdat, 800 mM Natriumchlorid und 50 U Glycerinoxidase in 0,2 M Kaliumphosphatpuffer, pH 6,8. In das Reaktionsgefäss ragt der Detektor 3, der aus einer sauerstoffsensitiven Elektrode besteht (WTW: OXI-Elektrode E 016). Der Detektor ist mit dem Analysator 4 verbunden (WTW: Digitalmeter DIGI 610 mit Einschub OXI 610 D). Am Boden des Reaktionsgefässes 1 befindet sich ein Magnetrührer 5.

Durch die Einfüllöffnung 6 werden 20 µl einer wässrigen glycerinhaltigen Lösung als Probe zugegeben. Während kräftigem Rühren mit dem Magnetrührer wird die Abnahme der Sauerstoffkonzentration der Lösung gemessen. Der beobachtete Sauerstoffverbrauch wird gegen die Glycerinkonzentration aufgetragen.

Beispiel 4
Kinetische Bestimmung

Proben: wässrige Standards (Glyceringehalt von 10 bis 100 mg/dl)
Reagens: 0,1 Mol/l Pipes-Puffer, 1,7 Mol/l $H_3BO_3$, pH = 7,0
3,0 g/l Isotridecyläther
3,0 g/l Natriumcholat
0,5 mMol/l 4-Aminoantipyrin, aminosubstituiert
10,0 mMol/l p-Chlorphenol
10,0 KU/l Peroxidase
121,0 KU/l Glycerinoxidase

Durchführung am Gemsaec-Fast-Analyzer-Analysenautomaten

| | |
|---|---|
| Temperatur: | 25° C |
| Messwellenlänge: | 546 nm |
| Probenvolumen: | 10 µl |
| Verdünnungsmittel ($H_2O$): | 50 µl |
| Reagensvolumen: | 500 µl |

Messzeit: 1. Ablesung: 35 Sek. nach Start
2. Ablesung: 315 Sek. nach Start.

Folgende Ergebnisse wurden erhalten:
Proben: wässrige Standards

| Glycerin (UV-Test) (mg/dl) | % Wiederfindung (Glyc-OD, erfindungsgemäss |
|---|---|
| 11 | 105 |
| 21 | 101 |
| 32 | 100 |
| 43 | 101 |
| 54 | 100 |
| 65 | 99 |
| 76 | 99 |
| 87 | 99 |
| 96 | 102 |

Vorzugsweise wird bei kinetischer Bestimmung mit folgenden Mengenverhältnissen im Reagens gearbeitet:
0,05 bis 0,5 Mol/l Pipes-Puffer, 0,5 bis 2,0 Mol/l $H_3BO_3$, pH 6,8 bis 8,0
1 bis 5 g/l Isotridecyläther
1 bis 5 g/l Natriumcholat
0,2 bis 5 mMol/l 4-Aminoantipyrin, aminosubstituiert
5 bis 50 mMol/l p-Chlorphenol
5 bis 50 KU/l Peroxidase
100 bis 250 KU/l Glycerinoxidase

Das Beispiel zeigt, dass die Erfindung an den wichtigsten, in einem klinisch-chemischen Routinelabor vorhandenen Analysenautomaten ausführbar ist.

**Patentansprüche**

1. Verfahren zur Bestimmung von Glycerin durch Oxidation mit Sauerstoff in Gegenwart von Glycerinoxidase und Messung des Sauerstoffverbrauchs oder der $H_2O_2$-Bildung, dadurch gekennzeichnet, dass man eine Glycerinoxidase aus Aspergillus spec. DSM 1729 verwendet.

2. Verfahren nach Anspruch 1, dadurch gekennzeichnet, dass der Sauerstoffverbrauch polarometrisch gemessen wird.

3. Verfahren nach Anspruch 1, dadurch gekennzeichnet, dass gebildetes $H_2O_2$ enzymatisch mit Katalase oder Peroxidase bestimmt wird.

4. Reagens zur Bestimmung von Glycerin, dadurch gekennzeichnet, dass es aus Glycerinoxidase aus Aspergillus spec. DSM 1729 und einem System zur Bestimmung von $H_2O_2$ besteht.

5. Reagens nach Anspruch 4, dadurch gekennzeichnet, dass es zusätzlich ein Mittel zur Verseifung von verestertem Glycerin enthält.

6. Reagens nach Anspruch 4 oder 5, dadurch gekennzeichnet, dass das System zur Bestimmung von $H_2O_2$ aus Katalase, Acetylaceton, Methanol und Puffer besteht.

7. Reagens nach Anspruch 4 oder 5, dadurch gekennzeichnet, dass das System zur Bestimmung von $H_2O_2$ aus Peroxidase, mindestens einem Chromogen und Puffer besteht.

8. Reagens nach Anspruch 7, dadurch gekennzeichnet, dass es 2 bis 150 U/ml Glycerinoxidase, 0,5 bis 100 U/ml Peroxidase, 0,05 bis 20 mMol/l Chromogen und gegebenenfalls 0,001 bis 0,1 g/ml wenigstens eines oberflächenaktiven Mittels oder ein Vielfaches davon und Puffer, pH 6 bis 9, enthält.

9. Reagens nach Anspruch 7, dadurch gekennzeichnet, dass es 100 bis 250 KU/l Glycerinoxidase, 5 bis 50 KU/l Peroxidase, 0,2 bis 5 mMol/l Chromogen und gegebenenfalls 1 bis 5 g/l wenigstens eines oberflächenaktiven Mittels oder ein Vielfaches davon und Puffer, pH 6,8 bis 8,0, enthält.

10. Reagens nach einem der Ansprüche 4 bis 9, dadurch gekennzeichnet, dass es auf einem Trägermaterial, wie Papier, imprägniert vorliegt.

11. Verfahren zur Gewinnung von Glycerinoxidase aus Aspergillus spec. DSM 1729, dadurch gekennzeichnet, dass man Aspergillus spec. DSM 1729 in einem geeigneten Nährmedium züchtet und die Glycerinoxidase aus der Zellmasse oder dem Kulturmedium gewinnt und gegebenenfalls reinigt.

12. Verfahren nach Anspruch 11, dadurch gekennzeichnet, dass man ein Medium verwendet, welches 20 bis 200 g/l Glycerin, 5 bis 100 g/l Malzextrakt, 0,5 bis 5 g/l Hefeextrakt, 0,2 bis 5 g/l Dikaliumphosphat, 0,2 bis 5 g/l Kaliumnitrat, 0,1 bis 5 g/l Calciumcarbonat, 0,1 bis 1 g/l Natriumchlorid, 0,001 bis 0,1 g/l Ferrosulfat (gemessen als Heptahydrat), 0,1 bis 5 g/l Magnesiumsulfat (gemessen als Heptahydrat) enthält.

13. Verfahren nach Anspruch 11 oder 12, dadurch gekennzeichnet, dass man die Zellmasse aufschliesst, vom Unlöslichen abtrennt und die Glycerinoxidase aus dem Überstand mit Trichloressigsäure fällt.

14. Verfahren nach Anspruch 13, dadurch gekennzeichnet, dass die Fällung bei pH 4,6 bis 5,4 durchgeführt wird.

15. Verfahren nach Anspruch 13 oder 14, dadurch gekennzeichnet, dass man anschliessend eine Acetonfällung, eine Ammonsulfatfraktionierung und eine Adsorption an einem schwach basischen Ionenaustauscher durchführt.

16. Glycerinoxidase aus Aspergillus spec. DSM 1729 mit einem Molekulargewicht von etwa 90 000, einer spezifischen Aktivität von 2000 bis 8000 U/mg, welche durch SH-Reagenzien gehemmt, durch Kupfersulfat und Bleiacetat nicht gehemmt wird.

**Claims**

1. Process for the determination of glycerol by oxidation with oxygen in the presence of glycerol oxidase and measurement of the oxygen consumption or of the $H_2O_2$ formation, characterised in that one uses a glycerol oxidase from Aspergillus spec. DSM 1729.

2. Process according to claim 1, characterised in that the oxygen consumption is measured polarometrically.

3. Process according to claim 1, characterised in that the $H_2O_2$ formed is determined enzymatically with catalase or peroxidase.

4. Reagent for the determination of glycerol, characterised in that it consists of glycerol oxidase from Aspergillus spec. DSM 1729 and a system for the determination of $H_2O_2$.

5. Reagent according to claim 4, characterised in that it additionally contains an agent for the saponification of esterified glycerol.

6. Reagent according to claim 4 or 5, characterised in that the system for the determination of $H_2O_2$ consists of catalase, acetylacetone, methanol and buffer.

7. Reagent according to claim 4 or 5, characterised in that the system for the determination of $H_2O_2$ consists of peroxidase, at least one chromogen and buffer.

8. Reagent according to claim 7, characterised in that it contains 2 to 150 U/ml. glycerol oxidase, 0.5 to 100 U/ml. peroxidase, 0.05 to 20 mMol/l. chromogen and possibly 0.001 to 0.1 g./ml. of at least one surface-active agent or a multiple thereof and buffer, pH 6 to 9.

9. Reagent according to claim 7, characterised in that it contains 100 to 250 KU/l. glycerol oxidase, 5 to 50 KU/l. peroxidase, 0.2 to 5 mMol/l. chromogen and possibly 1 to 5 g./l. of at least one surface-active agent or a multiple thereof and buffer, pH 6.8 to 8.0.

10. Reagent according to one of claims 4 to 9, characterised in that it is present impregnated on a carrier material, such as paper.

11. Process for the obtaining of glycerol oxidase from Aspergillus spec. DSM 1729, characterised in that one cultures Aspergillus spec. DSM 1729 in a suitable nutrient medium and obtains the glycerol oxidase from the cell mass or from the culture medium and possibly purifies.

12. Process according to claim 11, characterised in that one uses a medium which contains 20 to 200 g./l. glycerol, 5 to 100 g./l. malt extract, 0.5 to 5 g./l. yeast extract, 0.2 to 5 g./l. dipotassium phosphate, 0.2 to 5 g./l. potassium nitrate, 0.1 to 5 g./l. calcium carbonate, 0.1 to 1 g./l. sodium chloride, 0.001 to 0.1 g./l. ferrosulphate (measured as heptahydrate), 0.1 to 5 g./l. magnesium sulphate (measured as heptahydrate).

13. Process according to claim 11 or 12, characterised in that one digests the cell mass, separates off from insolubles and precipitates the glycerol oxidase from the supernatant with trichloroacetic acid.

14. Process according to claim 13, characterised in that the precipitation is carried out at pH 4.6 to 5.4.

15. Process according to claim 13 or 14, characterised in that one subsequently carries out an acetone precipitation, an ammonium sulphate fractionation and an adsorption on a weakly basic ion exchanger.

16. Glycerol oxidase from Aspergillus spec. DSM 1729 with a molecular weight of about 90,000, a specific activity of 2000 to 8000 U/mg., which is inhibited by SH reagents but is not inhibited by copper sulphate and lead acetate.

**Revendications**

1. Procédé pour le dosage du glycérol par oxydation par l'oxygène en présence de glycéroloxydase et mesure de la consommation d'oxygène ou de la formation de $H_2O_2$, caractérisé en ce qu'on utilise une glycéroloxydase provenant d'Aspergillus spec. DSM 1729.

2. Procédé selon la revendication 1, caractérisé en ce que la consommation d'oxygène est mesurée polarométriquement.

3. Procédé selon la revendication 1, caractérisé en ce que le $H_2O_2$ formé est mesuré enzymatiquement au moyen de catalase ou de peroxydase.

4. Réactif pour le dosage du glycérol, caractérisé en ce qu'il est constitué de glycéroloxydase provenant d'Aspergillus spec. DSM 1729 et d'un système pour le dosage de $H_2O_2$.

5. Réactif selon la revendication 4, caractérisé en ce qu'il contient en outre un agent pour saponifier le glycérol estérifié.

6. Réactif selon la revendication 4 ou 5, caractérisé en ce que le système pour le dosage de $H_2O_2$ est constitué de catalase, d'acétylacétone, de méthanol et de tampon.

7. Réactif selon la revendication 4 ou 5, caractérisé en ce que le système pour le dosage de $H_2O_2$ est constitué de peroxydase, d'au moins un chromogène et de tampon.

8. Réactif selon la revendication 7, caractérisé en ce qu'il contient 2 à 150 U/ml de glycéroloxydse, 0,5 à 100 U/ml de peroxydase, 0,05 à 20 mmoles/l de chromogène et éventuellement 0,001 à 0,1 g/ml d'au moins un agent tensio-actif ou un multiple de ceci et du tampon, pH 6 à 9.

9. Réactif selon la revendication 7, caractérisé en ce qu'il contient 100 à 250 KU/l de glycéroloxydase, 5 à 50 KU/l de peroxydase, 0,2 à 5 mmoles/l de chromogène et éventuellement 1 à 5 g/l d'au moins un agent tensioactif ou un multiple de ceci et du tampon, pH 6,8 à 8,0.

10. Réactif selon l'une des revendications 4 à 9, caractérisé en ce qu'il se présente imprégné sur une matière support telle que du papier.

11. Procédé pour l'obtention de glycéroloxydase à partir d'Aspergillus spec. DSM 1729, caractérisé en ce qu'on cultive Aspergillus spec. DSM 1729 dans un milieu nutritif approprié et en ce qu'on recueille la glycéroloxydase à partir de la masse cellulaire ou du milieu de culture et éventuellement purifie.

12. Procédé selon la revendication 11, caractérisé en ce qu'on utilise un milieu qui contient 20 à 200 g/l de glycérol, 5 à 100 g/l d'extrait de malt, 0,5 à 5 g/l d'extrait de levure, 0,2 à 5 g/l de phosphate dipotassique, 0,2 à 5 g/l de nitrate de potassium, 0,1 à 5 g/l de carbonate de calcium, 0,1 à 1 g/l de chlorure de sodium, 0,001 à 0,1 g/l de sulfate ferreux (mesuré en tant qu'heptahydrate), 0,1 à 5 g/l de sulfate de magnésium (mesuré en tant qu'heptahydrate).

13. Procédé selon la revendication 11 ou 12, caractérisé en ce qu'on désagrège la masse cellulaire, sépare des insolubles et fait précipiter la clycéroloxydase à partir du surnageant au moyen d'acide trichloroacétique.

14. Procédé selon la revendication 13, caractérisé en ce que la précipitation est effectuée à pH 4,6 à 5,4.

15. Procédé selon la revendication 13 ou 14, caractérisé en ce qu'on effectue ensuite une précipitation à l'acétone, un fractionnement au sulfate d'ammonium et une adsorption sur un échangeur d'ions faiblement basique.

16. Glycéroloxydase provenant d'Aspergillus spec. DSM 1729 avec un poids moléculaire d'environ 90 000, une activité spécifique de 2000 à 8000 U/mg, laquelle est inhibée par les réactifs SH, n'est pas inhibée par le sulfate de cuivre et l'acétate de plomb.

FIG.1

FIG. 2

ΔE 546nm

GLYCERIN - KONZENTRATION ( mg/ml )